# DEMANDE DE BREVET EUROPEEN

(11) **EP 1 559 417 A1**
(43) Date de publication de la demande: **03.08.2005**
(21) Numéro de dépôt: 05300079.0
(22) Date de dépôt: 01.02.2005
(51) Int. Cl.: A61K 7/48

(54) **Principe actif obtenu a partir de poudre de semences de Medicago sativa**

(30) Priorité: 02.02.2004 FR 0400986
(71) Demandeur: Société Industrielle Limousine d'Application Biologique Dite SILAB, 19130 Objat (FR)
(72) Inventeur: Paufique, Jean, 19130, OBJAT (FR)
(74) Mandataire: Fantin, Laurent

(57) **Abrégé**

L'objet de l'invention est un principe actif issu de poudre de semences d'alfalfa (Medicago saliva) permettant de lutter de manière similaire au rétinol, contre les phénomènes de vieillissement extrinsèques de la peau avec des propriétés anti-rides et lissantes.
L'invention couvre aussi le principe actif, le procédé d'obtention ainsi que les compositions l'incluant.

## Description

La présente invention couvre un principe actif issu de poudre de semences d'alfalfa en vue de moduler l'expression d'un grand nombre de protéines épidermiques, d'agir sur le renouvellement cellulaire des fibroblastes, de maintenir le bon fonctionnement cellulaire, de stimuler la synthèse du collagène I en inhibant l'action des enzymes responsables de sa dégradation.

L'invention couvre aussi le principe actif utilisé, en tant que tel ainsi que le procédé d'obtention.

Compte tenu de la durée de vie qui augmente, il convient de faire en sorte que l'esthétique, notamment du visage par lequel passe souvent le jugement des autres, soit en corrélation.

Les industries de la cosmétique cherchent à lutter contre le vieillissement cutané qui reste un processus complexe qui affecte toutes les couches de la peau.

Des études ont conduit à attribuer des proportions importantes au phénomène de vieillissement extrinsèque, près de 75%, en regard du vieillissement dit intrinsèque lié à la génétique de la personne, seulement 25%.

Les facteurs les plus importants sont les agressions extérieures, soleil, pollution, alimentation, mauvaise hygiène de vie en général, donc essentiellement les radicaux libres si l'on se place sur le plan moléculaire.

Plus généralement, les signes se traduisent par une diminution de la fonction barrière de la peau et un relâchement cutané, particulièrement engendrés par la perte de propriétés mécaniques de la peau. Les fibroblastes ont une activité métabolique ralentie. Les fibres d'élastine se dégradent. La synthèse de collagène, donc sa teneur, diminue dans le derme. La teneur en protéoglycanes diminue et il se produit une perte des propriétés viscoélastiques du derme.

Afin de lutter contre ces phénomènes, on connaît les activités du rétinol ou vitamine A et de ses dérivés sur les cellules de la peau. Les rétinoïdes jouent un rôle essentiel dans l'embryogenèse et le contrôle de la croissance cellulaire et leur apport par voie topique est utilisé pour le traitement de certaines maladies de peau.

Cette utilisation par voie topique améliore les caractéristiques des peaux âgées, agissant sur les rides, les ridules et/ou la couleur même de la peau.

Néanmoins, dans la famille des rétinoïdes, il a été constaté des effets secondaires provoqués par l'usage de l'acide rétinoïque. Notamment, cet acide est fortement irritant, interdisant son application en cosmétique.

Quant au rétinol, il est fortement instable à la lumière, à l'oxygène et à la chaleur, et surtout très irritant. Le rétinaldéhyde ou ses formes esters sont plus stables mais pénètrent moins aisément dans la peau.

Le but de la présente invention est de proposer un principe actif qui présente des effets du type de ceux du rétinol sans en présenter les inconvénients et les effets secondaires. L'invention couvre aussi le procédé d'obtention et les compositions l'incluant.

Comme pour les rétinoïdes, le principe actif selon l'invention vise à augmenter l'effet barrière du stratum corneum et à moduler l'expression d'un grand nombre de protéines épidermiques.

Ainsi, le principe actif agit sur le renouvellement cellulaire des fibroblastes et participe au maintien du fonctionnement de la matrice extracellulaire.

Le principe actif stimule la synthèse du collagène I tout en inhibant l'action des enzymes responsables de sa dégradation.

Plus particulièrement, le principe actif influence de façon positive la différenciation kératinocytaire à travers différents marqueurs connus tels que :
- cytokératine 4,
- cytokératine 19,
- HSP 27, et
- profilaggrine.

Le procédé selon l'invention consiste à recourir à des semences d'alfalfa (Medicago sativa) comme base naturelle d'obtention. Ces semences renferment un fort taux de sucres, d'oligo-éléments et d'acides aminés.

Dans le procédé selon l'invention :
- on solubilise de la poudre de semences d'alfalfa (Medicago saliva) à raison d'au moins 50g/l dans un milieu aqueux,
- on hydrolyse de façon simultanée et/ou consécutive cette préparation en présence de protéases et de carbohydrases,
- on sépare les fractions soluble et insoluble,
- on inactive les enzymes, et
- on concentre la fraction active par tout moyen adapté tel que la décantation, la filtration, l'osmose, l'osmose inverse ou la nanofiltration.

### 1/ PRINCIPE ACTIF :

On obtient par ce procédé un principe actif qui peut être caractérisé par différents paramètres.

### 1-1/ Taux de matière sèche :

On utilise un échantillon placé dans une étuve à 105°C jusqu'à obtention d'un poids constant.

Le taux de matière sèche obtenu est de 10 à 200 g/l, préférentiellement de 40 à 55 g/l.

### 1-2/ Teneur en sucres totaux :

On utilise la méthode de DUBOIS (DUBOIS M. & al [1956], Analytical chemistry, 28, n°3 p 350-356).

En présence d'acide sulfurique concentré et de phénol, les sucres réducteurs donnent un composé jaune-orangé.

A partir d'une gamme étalon de mannose, glucose, galactose, allant de 25 µg/l à 100 µg/l, on peut déterminer le taux de sucres totaux d'un échantillon.

Le taux de sucres totaux du principe actif selon la présente invention est de 4 à 90 g/l, préférentiellement de 17 à 25 g/l.

### 1-3/ Caractérisation des sucres simples :

Pour étudier les sucres simples, on détermine a) la quantité de chacun des sucres et b) le degré de polymérisation.
a) L'analyse HPLC conduit à la quantité suivante de carbohydrates présents dans le principe actif selon la présente invention :
   - 8,4 % de glucose,
   - 39,1 % de mannose, et
   - 52,5 % de galactose.
b) L'analyse du degré de polymérisation montre :
   - 41,7 % de monosaccharides avec un degré de polymérisation de 1,
   - 50,1 % de disaccharides avec un degré de polymérisation de 2,
   - 4,1 % d'oligosaccharides avec un degré de polymérisation de 5, et
   - 4,1 % d'oligosaccharides et de polysaccharides avec un degré de polymérisation supérieur à 7.

La fraction glucidique de ce principe actif obtenu à partir de semences d'alfalfa (Medicago sativa) est composé essentiellement de galactose, glucose et mannose sous forme de mono, di et oligosaccharides.

Plus particulièrement, on retrouve une composition de la fraction glucidique sous forme de monosaccharides et disaccharides pour au moins 90%, et sous forme d'oligosaccharides et polysaccharides pour le complément.

### 1-4/ Détermination de la fraction active de cette composition glucidique :

Les sucres de ce principe actif sont fractionnés par chromatographie gel filtration.

La détection est réalisée à 280 nm, le tampon d'élution étant un tampon phosphate et chlorure de sodium.

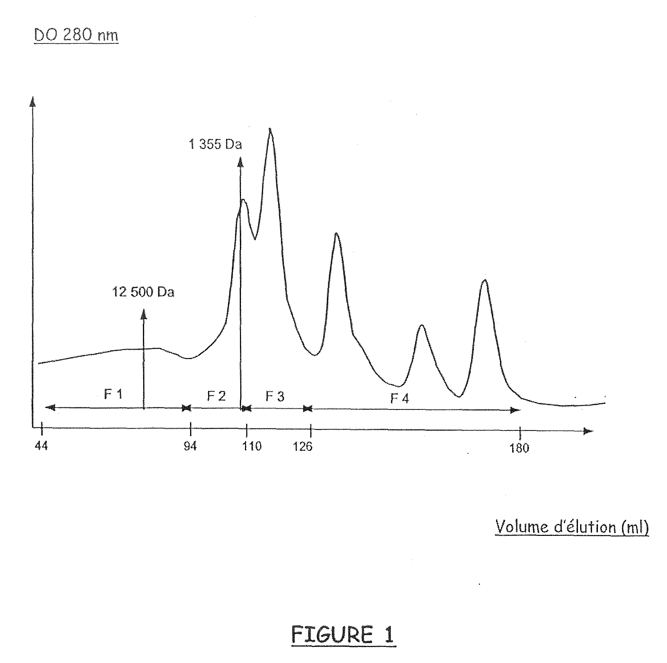

La figure 1 montre la courbe obtenue.

Les volumes d'élution des fractions sont les suivants :
- fraction F0, 0-44 ml
- fraction F1, 44-94 ml
- fraction F2, 94-110 ml,
- fraction F3,110-126 ml, et
- fraction F4, 126-180 ml.

### a/ Synthèse de collagène I :

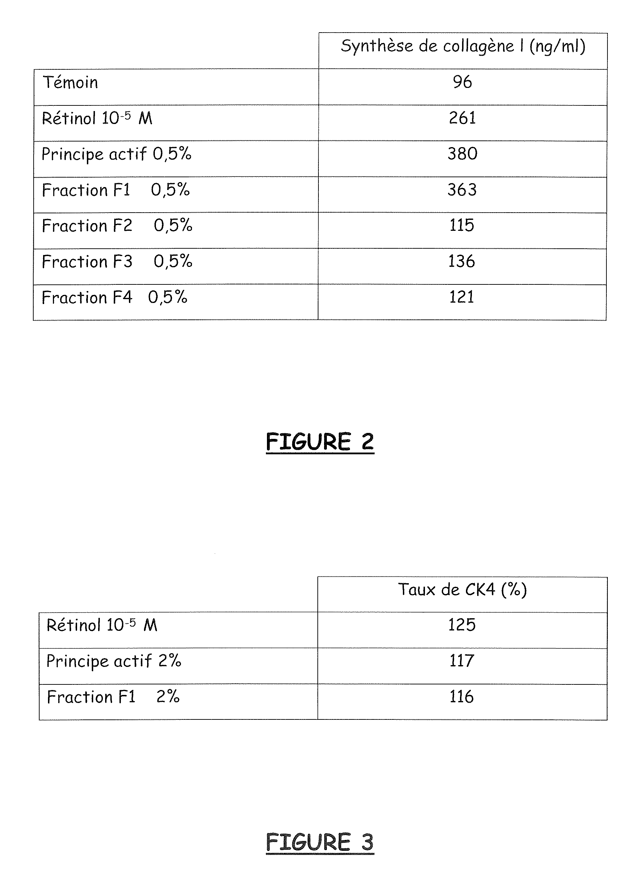

L'étude est conduite sur fibroblastes humains par dosage ELISA. Les fibroblastes sont incubés en présence ou non du principe actif, des fractions ou du rétinol à 10⁻⁵ M. Les résultats sont représentés sur la figure 2.

La fraction F1 est aussi active que le principe actif complet sur la synthèse du collagène I, respectivement 363 ng/ml et 380 ng/ml.

C'est la fraction F1 qui confère au principe actif son activité sur la synthèse du collagène I.

### b/ Synthèse de la cytokératine-4 :

L'objectif est de montrer que la fraction F1 identifiée comme la fraction active l'est aussi sur la différenciation kératinocytaire.

Le tableau de la figure 3 montre que l'effet du principe actif complet est dû à la fraction F1, et que cet effet est comparable à celui du rétinol.

C'est aussi la fraction F1 polysaccharidique qui confère au principe actif les propriétés recherchées.

### 1-5/ Analyse de la fraction active F1 :

Les oses neutres sont dosés dans le principe actif pris dans sa composition brute et dans la fraction active F1, par la méthode de DUBOIS référencée ci-avant.

Les résultats sont les suivants :
- principe actif brut : 20 g/l
- fraction active F1 : 5,9 g/l

Le détail de la composition en oses neutres est obtenu par analyse à l'aide de la méthode de Kamerling & al (1975) modifiée par Montreuil & al (1986). Le principe repose sur l'obtention de méthylglycosides triméthylsylilés par une méthanolyse suivie d'une pertriméthylsylilation à partir des monosaccharides constituant l'échantillon.

L'analyse est ensuite effectuée par Chromatographie en Phase Gazeuse.

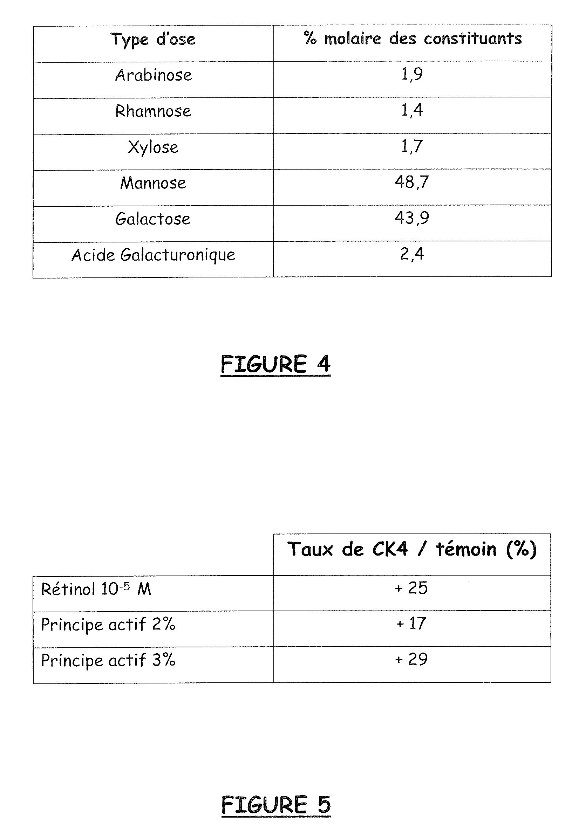

Les résultats obtenus sont indiqués dans le tableau de la figure 4.

On constate que la fraction active F1 est une fraction polysaccharidique riche en galactomannanes.

### 2/ IDENTIFICATION ET QUANTIFICATION DU PRINCIPE ACTIF EN FORMULE :

On cherche par cette étape à identifier et quantifier le principe actif dosé à 1% en émulsion.

La composition de cette émulsion est la suivante :
- octanoate de cetearyl (Lanol 1688) : 10%
- alcool d'arachidyl/alcool de behenyl/glucoside d'arachidyl (Montanov 202) :3%
- isononanoate isononyl (Lanol 99) : 2%
- polyacrylamide/isoparaffine C13-14/laureth-7 (Sepigel 305) : 2%
- principe actif : 1%
- phenonip : 0,5 %, et
- eau : quantité suffisante pour 100%

On déphase la crème émulsionnée pour récupérer la phase aqueuse qui est analysée par HPLC (chromatographie liquide à haute performance) avec comme marqueur utilisé du principe actif l'acide p-coumarique.

Les résultats donnent une quantification de surface de pic par rapport au marqueur de : 5,77/648,4 soit une concentration de 0,9% de principe actif dans la composition en émulsion.

On retrouve bien le principe actif formulé à 1% dans sa quasi intégralité.

### 3/ EFFICACITE COSMETIQUE IN VITRO :

### 3-1/ Différenciation kératinocytaire :

### Synthèse des cytokératines 4 et 19 :

L'étude porte sur des kératinocytes humains. Une partie est traitée avec le principe actif selon l'invention et l'autre partie est traitée avec du rétinol à des fins de comparaison.

Les protéines totales sont ensuite dosées par la méthode BCA (Sigma).

On réalise une électrophorèse des extraits cellulaires ramenés à la même quantité de protéines sur un gel. On transfère les protéines sur une membrane.

On réalise alors un immunomarquage des deux types de cytokératine avec des anticorps adaptés.

On constate alors les effets du principe actif dosé à 2% et 3%, sur la synthèse des cytokératines 4 et 19 comparés à ceux engendrés par le rétinol.

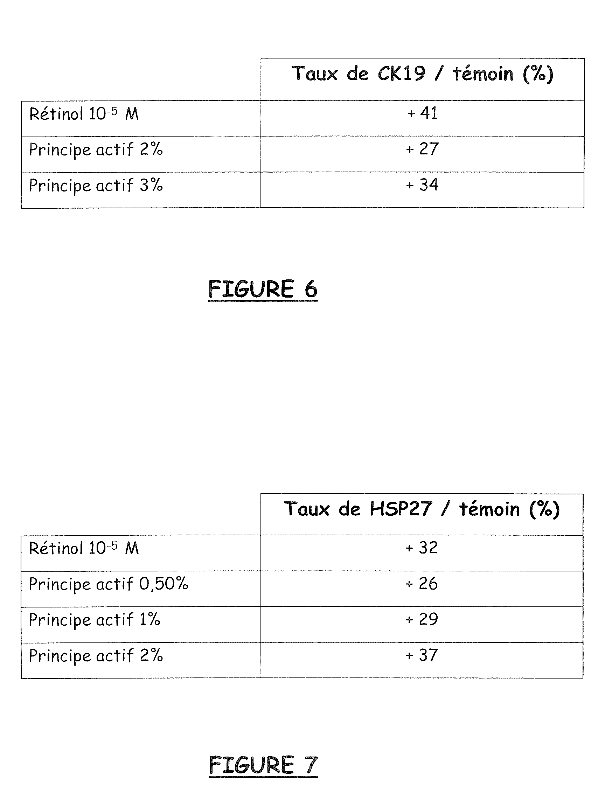

Les résultats sont indiqués dans les tableaux des figures 5 et 6 respectivement.

On constate que les résultats sont sensiblement identiques à ceux obtenus par le rétinol montrant ainsi que le principe actif extrait de l'alfalfa augmente significativement la synthèse des cytokératines 4 et 19 particulièrement.

### Synthèse des HSP 27 (Heat Schock Protein) :

Cette protéine est un marqueur du processus de différenciation kératinocytaire.

L'étude est réalisée sur des kératinocytes humains traités avec le rétinol comme base de comparaison et avec le principe actif dosé à 0,5%, 1% et 2%

On met en oeuvre le même protocole que précédemment avec les immunomarqueurs et les révélateurs adaptés.

On obtient les résultats regroupés en figure 7.

Le principe actif selon la présente invention permet de favoriser significativement la synthèse des HSP 27 de 26% dès 0,5%.

### Synthèse de la profilaggrine :

La profilaggrine est aussi impliquée dans le processus de différenciation. Notamment la profilaggrine est transformée en filaggrine, molécule qui permet l'agrégation des filaments de cytokératine.

On sait que le rétinol stimule la synthèse de cette protéine dans les conditions de cette étude.

Le protocole qui va suivre permet de déterminer les capacités du principe actif issu d'alfalfa à stimuler également la synthèse de cette protéine.

On incube des kératinocytes humains en présence du principe actif à 0,5%, 1% et 2%.

Les cellules sont récupérées et les ARN totaux sont extraits. Après analyse, on obtient les pourcentages d'expression de l'ARNm de profilaggrine par rapport au témoin.

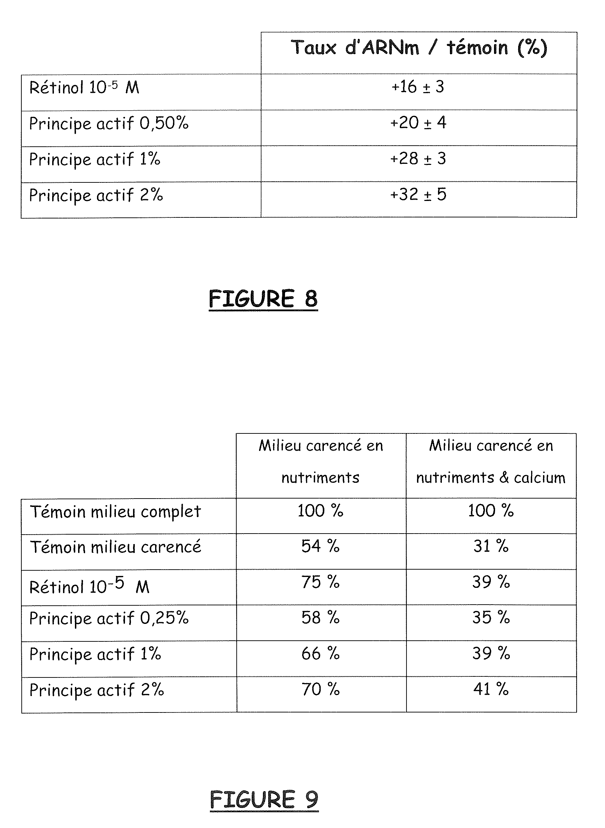

Les résultats sont indiqués dans le tableau de la figure 8.

On constate une stimulation sur la synthèse de profilaggrine de kératinocytes humains au moins comparable à celle du rétinol à 10⁻⁵ M.

### 3-2/ Influence sur le métabolisme des cellules du derme.:

### Effet sur la prolifération cellulaire de fibroblastes humains :

L'étude consiste à comparer la capacité du principe actif à favoriser la réplication et la prolifération de fibroblastes humains avec celle du rétinol.

On incube des fibroblastes humains soit dans un milieu carencé en calcium et complémenté en facteurs de croissance, soit dans un milieu carencé en calcium et non complémenté en facteurs de croissance.

On détermine le pourcentage de viabilité cellulaire et les résultats sont regroupés dans le tableau de la figure 9.

On constate que l'ajout de principe actif selon l'invention tend à restaurer une capacité de prolifération des fibroblastes en compensant les carences du milieu.

A 2%, on constate que le principe actif présente une activité de stimulation de la prolifération cellulaire semblable à celle du rétinol.

### Effet sur le métabolisme protéique :

Le but est d'évaluer l'effet du principe actif sur la synthèse du collagène I, composant majeur du derme.

Les tests sont réalisés sur des fibroblastes humains normaux par dosage ELISA. On traite les fibroblastes avec du rétinol à 10⁻⁵ M ou avec le principe actif dosé à 0,1% ; 0,25% et 0,5%.

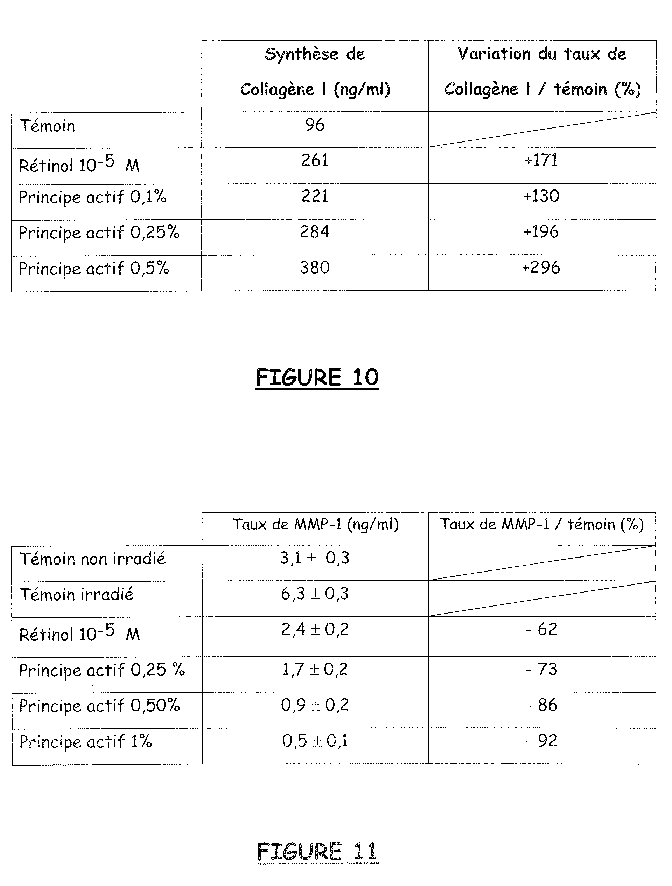

Les résultats récapitulés dans le tableau de la figure 10 montrent que le principe actif issu d'alfalfa stimule significativement la synthèse de collagène I de façon dose-dépendante, par exemple, une augmentation de 296 % pour un dosage à 0,5%. On note en comparaison que le rétinol à 10⁻⁵ M conduit à une augmentation de 171%.

### Effet sur l'activité anti-MMP-1 pour la protection des protéines de la matrice :

L'étude porte sur une culture de fibroblastes humains soumis à une exposition aux UVA. Le taux de MMP-1 augmente au niveau de la matrice extracellulaire en cas d'agression de ce type.

Les fibroblastes sont traités avec du rétinol à 10⁻⁵ M, ou traités avec le principe actif dosé à 0,25% ; 0,5% ; 1% ou encore non traités.

Les résultats sont récapitulés dans le tableau de la figure 11.

On constate que le principe actif inhibe la synthèse des MMP-1 de façon dose-dépendante. La capacité du principe actif est comparable à celle du rétinol.

### 3-3/ Effet du principe actif sur l'expression différentielle de gènes :

L'étude permet de comparer l'influence du principe actif à celle de l'acide rétinoïque sur l'expression différentielle des gènes dans des épidermes humains reconstruits par la méthode des "cDNA arrays".

Des épidermes sont traités avec de l'acide rétinoïque dosé à 10⁻⁶M ou avec le principe actif selon la présente invention dosé à 2%.

On étudie ensuite les effets de chacun des composés sur l'expression de gènes sélectionnés pour leur rôle vis-à-vis des cellules et de la matrice extracellulaire, des cytokératines ou vis-à-vis de certaines protéines d'intérêt cosmétique.

La méthode utilisée permet de constater que les gènes reportés comme étant modulés par l'acide rétinoïque le sont aussi par le principe actif selon l'invention, notamment à 2%.

Le principe actif issu de semences d'alfalfa induit l'expression d'un profil de gène très similaire à celui de l'acide rétinoïque.

### 4/ EFFICACITE COSMETIQUE IN VIVO :

### 4-1/ Effet anti-rides :

Le principe actif est formulé à 4% en émulsion. Le rétinol est formulé à 0,15% dans une émulsion.

Les 2 formules sont testées sur 2 groupes de volontaires contre placebo.

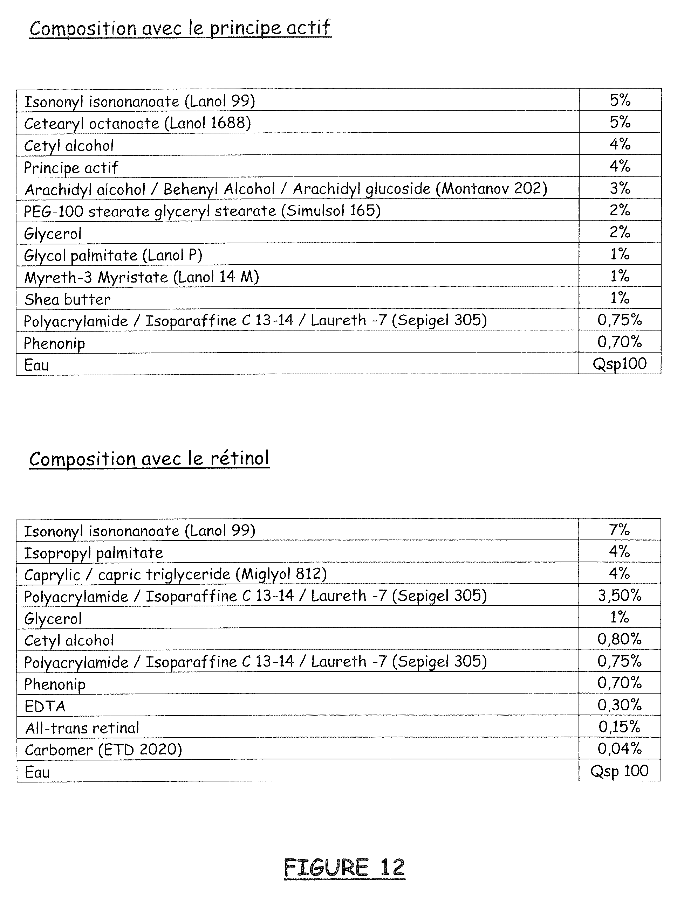

Les résultats obtenus sont regroupés dans les tableaux de la figure 12.

Les tests ont porté sur l'influence du principe actif ou du rétinol sur l'aspect des rides de la patte d'oie, après application biquotidienne pendant 28 jours.

Les analyses sont réalisées par observation d'empreintes à l'aide d'un profilomètre associé à un analyseur d' images.

Les paramètres obtenus sont :
- surface totale ridée,
- longueur totale de rides, et
- profondeur des rides.

Les deux compositions appliquées sont mentionnées à titre d'exemple dans les deux tableaux de la figure 12.

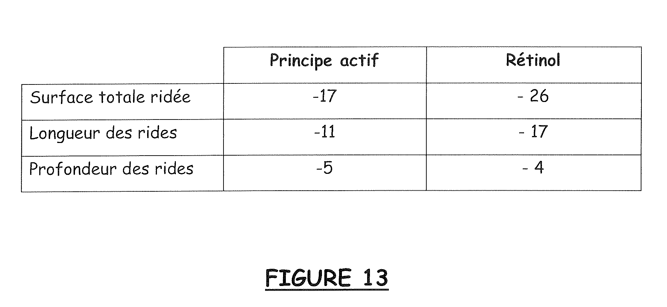

On obtient les résultats du tableau de la figure 13.

On constate que le principe actif présente des propriétés anti-rides comparables à celles obtenues avec le rétinol mais sans provoquer de réactions irritatives.

### 4-2/ Effet lissant :

Le principe actif est formulé à 4% en émulsion, le rétinol est formulé à 0,15% en émulsion.

Les 2 formules sont testées sur 2 groupes de volontaires contre placebo.

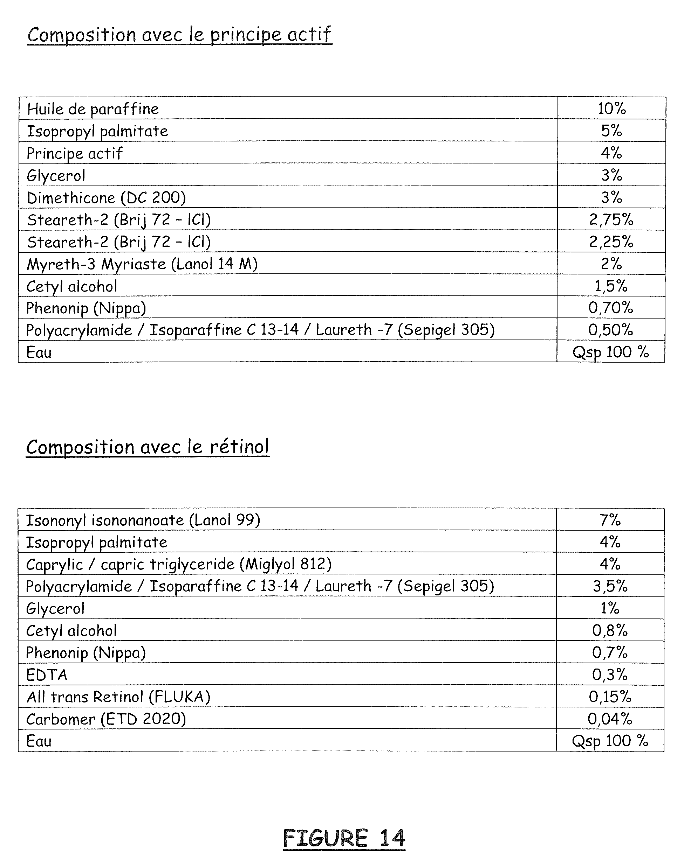

Les formules testées sont regroupées dans les tableaux de la figure 14.

Les tests ont porté sur l'influence du principe actif ou du rétinol sur l'aspect du microrelief cutané, après application biquotidienne pendant 28 jours.

Les analyses sont réalisées par observation d'empreintes du microrelief cutané à l'aide d'un profilomètre associé à un analyseur d'images.

Les zones concernées par cette étude sont des zones cutanées déterminées au niveau des avant bras. Le paramètre observé est la rugosité moyenne du microrelief.

Les résultats montrent un effet lissant du principe actif sur le micro relief de 8% par rapport au placebo pour le rétinol et de 6% par rapport au placebo pour le principe actif selon l'invention.

Par contre, comme indiqué en préambule, on note des réactions du type dermite irritative pour des volontaires ayant appliqué le rétinol provoquant la sortie prématurée de l'étude.

Le principe actif issu de semences d'alfalfa (Medicago sativa) est particulièrement actif puisque les différents tests et résultats indiqués ci-avant montrent des résultats similaires à ceux obtenus avec le rétinol tout en supprimant les effets secondaires indésirables du rétinol.

L'invention concerne de façon générale les compositions cosmétiques incluant le principe actif selon l' invention, à raison de 0,1 à 20% dans une forme galénique adaptée telle qu'une émulsion grasse ou aqueuse, une crème, une lotion, un onguent.

## Revendications

1. Principe actif issu de poudre de semences d'alfalfa (Medicago sativa) et principalement la fraction riche en galactomannanes permettant de lutter contre les phénomènes de vieillissement extrinsèques de la peau avec des propriétés anti-rides et lissantes.

2. Principe actif issu de poudre de semences d'alfalfa (Medicago sativa) et principalement la fraction riche en galactomannanes selon la revendication 1, **caractérisé en ce qu'**il présente une activité sur la synthèse du collagène I.

3. Principe actif issu de poudre de semences d'alfalfa (Medicago sativa) et principalement la fraction riche en galactomannanes selon la revendication 1, **caractérisé en ce qu'**il augmente la synthèse des cytokératines 4 et 19.

4. Principe actif issu de poudre de semences d'alfalfa (Medicago sativa) et principalement la fraction riche en galactomannanes selon la revendication 1, **caractérisé en ce qu'**il favorise la synthèse des HSP 27 (Heat Schock Protein) protéines, marqueur du processus de différenciation kératinocytaire.

5. Principe actif issu de poudre de semences d'alfalfa (Medicago sativa) et principalement la fraction riche en galactomannanes selon la revendication 1, **caractérisé en ce qu'**il stimule la synthèse de profilaggrine, impliquée dans le processus de différenciation kératinocytaire.

6. Principe actif issu de poudre de semences d'alfalfa (Medicago sativa) et principalement la fraction riche en galactomannanes selon la revendication 1, **caractérisé en ce qu'**il présente une capacité à inhiber l'activité enzymatique de la MMP-1, métalloprotéinase responsable de la dégradation des fibres de collagène I.

7. Principe actif issu de poudre de semences d'alfalfa (Medicago sativa) et principalement la fraction riche en galactomannanes selon la revendication 1,
**caractérisé en ce qu'**il présente une activité de stimulation de la prolifération cellulaire.

8. Principe actif issu de poudre de semences d'alfalfa (Medicago sativa) et principalement la fraction riche en galactomannanes, ayant les propriétés des revendications 1 à 7, **caractérisé par** les paramètres suivants :
- taux de matière sèche : 10 à 200 g/l,
- teneur en sucres totaux : 4 à 90 g/l
- sucres simples : 8,4 % de glucose,
39,1 % de mannose, et
52,5 % de galactose.
sous forme de mono, di et oligosaccharides et polysaccharides.

9. Principe actif selon la revendication 8, **caractérisé par** les paramètres suivants :
- taux de matière sèche : 40 à 55 g/l,
- teneur en sucres totaux : 17 à 25 g/l
- sucres simples : 8,4 % de glucose,
39,1 % de mannose, et
52,5 % de galactose.
sous forme de monosaccharides et disaccharides pour au moins 90%, et sous forme d'oligosaccharides et polysaccharides pour le complément.

10. Principe actif selon la revendication 8 ou 9, **caractérisé en ce que** la fraction active est la fraction polysaccharidique riche en galactomannanes.

11. Procédé d'obtention du principe actif selon l'une des revendications 8 à 10, **caractérisé en ce qu'**il comprend les étapes suivantes :
- on solubilise de la poudre de semences d'alfalfa (Medicago sativa) à raison d'au moins 50g/l dans un milieu aqueux,
- on hydrolyse de façon simultanée et/ou consécutive cette préparation en présence de protéases et de carbohydrases,
- on sépare les fractions soluble et insoluble,
- on inactive les enzymes, et
- on concentre la fraction active par tout moyen adapté tel que la décantation, la filtration, l'osmose, l'osmose inverse ou la nanofiltration.

12. Composition cosmétique incluant le principe actif selon l'une des revendications 8 à 10, **caractérisée en ce qu'**elle comprend ce principe actif à raison de 0,1 à 20% dans une forme galénique adaptée telle qu'une émulsion grasse ou aqueuse, une crème, une lotion, un onguent.
